# EUROPEAN PATENT SPECIFICATION

(11) **EP 0 575 535 B1**
(45) Date of publication and mention of the grant of the patent: **25.09.1996**
(21) Application number: 92909001.7
(22) Date of filing: 09.03.1992
(51) Int. Cl.: C09K 3/00, A61K 31/74, A61K 6/00, A61K 7/16

(54) **DENTAL COMPOSITION**
DENTALMASSE
COMPOSITION DENTAIRE

(30) Priority: 14.03.1991 US 669076; 19.06.1991 US 717886
(43) Date of publication of application: 29.12.1993
(73) Proprietor: ESSENTIAL DENTAL SYSTEMS, INC., South Hackensack, NJ 07606 (US)
(72) Inventor: COHEN, Brett, I., Nanuet, NY 10954 (US); MUSIKANT, Barry, L., New York, NY 10024 (US)
(74) Representative: Lamb, John Baxter
(86) International application number: US9201949
(87) International publication number: WO9216594

(56) References cited:
- US-A- 4 206 102

## Description

### Background of the Invention

This is a continuation-in-part of application Serial No. 669,076, filed March 14, 1991, now abandoned.

This invention relates to a dental composition, and more particularly, to a composition which is applied to dentures and other types of artificial teeth as well as to dental implants. The composition is used for preventing the build-up of plaque and other debris.

One of the major problems with dentures and other artificial teeth is that they quickly accumulate plaque and other debris along the surface of the denture material. This is obviously not desirable since wearer comfort is reduced, distasteful odors are produced and denture wearability is decreased.

There are a number of commercial compositions on the marketplace which may be used for cleaning dentures. These compositions include Efferdent, Polident and Dentu-Creme, and are used on or applied to the dentures when outside the mouth of the wearer. However, such compositions are less than satisfactory. These composition are not totally efficient and may not fully clean the dentures because of the excessive build-up of plaque and debris. In some cases, a toothbrush or other manual cleaner may be needed.

One of the recurring problems with dental implants is also the accumulation of plaque, particularly at the gingival level. To be removed, the dentist must take a Teflon coated instrument and scrape the material away. This results in scratches along the surface of the implant and also promotes further plaque accumulation in the future.

In the medical field, there are chemical formulations that have been used to form a coating along the surface of medical devices such as catheters. This type of coating provides a dry lubricated surface which reduces friction and drag. As a result, performance of these devices is enhanced. These chemical formulations have in the past comprised a ketone based composition that includes N-methylpyrrolidinone, a polyurethane resin and polytetrafluor-ethylene (Teflon).

In view of the previous success of this composition in forming a coating surface on medical equipment which exhibits reduced friction and drag, it would be desirable to determine whether the composition has application in the dental field, specifically as a coating for a denture material or dental implant.

According to the invention, there is provided a method for preventing accumulation or build up of plaque and other debris on dentures or other artificial teeth and dental implants comprising applying to the artificial teeth or implant a composition containing:
a solvent in an amount between 46 and 98 weight percent;
a volatile compound in an amount between 0.5 and 25 weight percent;
a polyurethane resin in an amount between 1 and 35 weight percent; and
a poly(fluoro) compound in an amount between 0.5 and weight percent.

The composition of the invention is prepared by mixing the N-methylpyrrolidinone, the polyurethane resin and the poly(fluoro) compound in the solvent until the chemicals dissolve and a homogeneous material is produced.

For application to dentures, the composition of the invention is typically sprayed on all external surfaces of the dentures and then allowed to dry.

For application to a dental implant, the composition is applied by dipping the implant in the composition (a solution) and then allowing the implant to dry.
Alternatively, the composition may be applied to the implant by painting.

### Detailed Description of the Preferred Embodiment

The composition of the invention includes a solvent in an amount between 46 and 98 weight percent. The solvent may be chosen from water, alcohols and ketones.

Suitable alcohols include ethanol, methanol, isopropyl alcohol, 3-pentanol, 2-pentanol, 1-pentanol, 1-hexanol, 2-hexanol, 3-hexanol and isobutyl alcohol. The preferred alcohol is ethanol in an amount between about 50 and 97 weight percent.

Suitable ketones include methylethyl ketone, acetone, allylacetone, isopropyl acetone, methylpropyl ketone, 3-pentanone, 3-hexanone and 2-hexanone. The preferred ketone is methylethyl ketone in an amount between about 50-97 weight percent.

The preferred amount of water (if chosen as the solvent) -n the inventive denture composition is in an amount between 50 and 97 weight percent.

In order to have a rapid mechanism for drying, the composition of the invention includes a volatile compound in an amount between 0.5 and 25 weight percent. The volatile compound is chosen from a pyrrolidone based or an ether based material. The preferred compound is a pyrrolidone based material chosen from N-methylpyrrolidinone (NMP), 2-pyrrolidone, 2-pyrrolidoneacetamide, l-ethyl-2-pyrolidinone and 5-methyl-2-pyrrolidinone. -N-methylpyrrolidinone is the preferred pyrrolidone material and may be present in the composition in an amount between 1 and 20 weight percent.

If an ether based material is instead chosen as the volatile compound of the inventive composition, it could be chosen from diethylether, isopropylether and pentylether.

As stated above, the purpose of including a volatile compound such as N-methylpyrrolidinone in the composition is to promote rapid drying of the composition when applied to either a denture material or a dental implant. This is achieved by accelerating evaporation at room temperature, thereby leaving a coating on the denture material or dental implant.

The inventive composition also includes a polyurethane resin in an amount between 1 and 35 weight percent. If the solvent chosen is either water or an alcohol, a water-based aliphatic polyurethane resin is chosen and is preferably present in an amount between 2 and 25 weight percent. If instead a ketone is used as a solvent, a non-water-based aromatic polyurethane resin is used and is preferably present in an amount between 2 and 25 weight percent.

Suitable water-based aliphatic polyurethane resins include adducts of 1,6-hexane diisocyanate (HDI) and isophorone diisocynate (IPDI), each with any of the following polyols: polyethylene oxide (PEO), polypropylene oxide (PPO), polyiosbutylene (PIB) and polytetramethylene oxide (PTMO). Therefore, a water-based polyurethane resin could be HDI/PPO, IPDI/PTMO or any other combination with an alphatic diisocyanate and a polyol group.

Suitable non-water based aromatic polyurethane resins include adducts of 2,4 toluene diisocyanate (2,4 TDI), 2,6 toluene diisocyanate (2,6 TDI), methylene bis (p-phenylisocyanate) (MDI), and 1,5-naphathalene diisocyanate (NDI), each with either of the following polyols: polyethylene oxide (PEO), polypropylene oxide (PPO), polyiosbutylene (PIB) and polytetramethylene oxide (PTMO). Therefore, a non-water based polyurethane resin could be 2,4 TDI/PPO, 2,4 TDI/PTMO, MDI/PPO, MDI/PEO, NDI/PEO or any other combination with an aromatic diisocyanate and a polyol group.

In addition, the polyurethane resin could also have a chain extender group such as ethylene glycol (EG), hexanediol (HD), 4,4 methylene bis (2-chloroaniline) (MOCA) and ethylene diamine (ED). Therefore, the polurethane resin can be HDI/ED/PPO and IPDI/EG/ PTMO (water based polyurethanes), or 2,4 TDI/ED/PPO, 2,4 TDI/MOCA/PTMO, MDI/EG/PPO: MDI/ED/PEO and NDI/ED/- PEO (non-water based polyurethanes).

The purpose of including a polyurethane resin in the inventive composition is to provide a hard, resistant coating (a chemical matrix) in order to "lock in" the Teflon on the applied surface of the denture or dental implant. It also increases wearability of the material to which the inventive composition is applied.

The inventive dental composition further includes a poly(fluoro) compound such as a fluorinated hydrocarbon in an amount between 0.5 and 20 weight. The preferred fluorinated hydrocarbon is polytetra-fluorethylene (Teflon) in an amount between 0.5 and 15 weight percent. The purpose of including Teflon in the composition is to prevent the build-up of plaque and other debris and dirt on the surfaces of the denture or dental implant, and also to reduce drag and resistance under normal wear.

Instead of using polytetra-fluorethylene in the composition, the composition may include as a fluorinated hydrocarbon polychlorotrifluoroethylene, polyhexafluoropropylene, polyvinylidine fluoride, polyvinylfluoride, copolymer mixtures of tetrafluoroethylene and ethylene, and mixtures of tetrafluoroethylene, propylene and flourinated copolymers of ethylene propylene.

In addition to the above identified ingredients, the composition of the invention may also include flavors (artificial or natural) and colorants.

In order to prepare the composition of the invention, the volatile compound, polyurethane resin and poly(fluoro) compound are dissolved in the solvent until the system is homogenous. Heat may be applied to facilitate dissolution.

For application to dentures, the composition should include a solvent in an amount between 66 and 98 weight percent, a volatile compound such as N-methylpyrrolidinone in an amount between 0.5 and 15 weight percent, a polyurethane resin in an amount between 1 and 25 weight percent and a poly(fluoro) compound such as polytetra-fluorethylene (Teflon) in an amount between 0.5 and 20 weight percent.

Preferably, the solvent is present in an amount between 75 and 97 weight percent. The preferred volative compound is N-methylpyrrolidinone in an amount between 1 and 8 weight percent.

If the solvent is either water or an alcohol, as stated hereinabove, a water based aliphatic polyurethane resin is chosen and is present in an amount between 2 and 15 weight percent. If instead a ketone is used as a solvent, a non-water based aromatic resin is chosen and is present in an amount between 2 and 20 weight percent.

Polytetra-fluoroethylene is the preferred hydrocarbon and is present in an amount between 0.5 and 3 weight percent.

In order to better comprehend the composition of the invention as applied to dentures and other artificial teeth, the following preferred examples are provided.

### Example 1

| | |
|---|---|
| Water | 96 weight percent (96 grams) |
| N-methyl-pyrrolidinone | 1 weight percent (1 gram) |
| Polyurethane | 2.4 weight percent (2.4 grams) |
| Polytetra-fluorethylene | 0.6 weight percent (0.6 grams) |

### Example 2

| | |
|---|---|
| Ethanol | 96 weight percent (96 grams) |
| N-methylpyrrolidinone | 1 weight percent (1 gram) |
| Polyurethane | 2.4 weight percent (2.4 grams) |
| Polytetra-fluorethylene | 0.6 weight percent (0.6 grams) |

In order to test the effectiveness of the preferred water based (Example 1) denture composition, the following experiment was conducted.

Ten acrylic disks (diameter 23mm, thickness 4mm) were coated on one side with the denture composition and allowed to dry for two hours (the other side was left the same and uncoated). The disks were then placed in a 2% solution of Methylene Blue (Basic Blue 9, C.I. 52015) (made up of 2 grams of Methylene Blue with 98 grams of water) in a 400 mL beaker and stirred for two hours.

After two hours, the disks were then removed from the methylene blue solution, rinsed with water and air dried. In all cases, the disk sides that were not coated with the denture solution were stained with the methylene blue. Removal of the methylene blue stain was not possible even by rubbing the disks with a paper towel (the blue stain was permanently fixed on the acrylic disk). The other sides of the disks with the denture coating of the invention remained unstained, thus demonstrating the effectiveness of the inventive denture composition.

In examples 1 and 2, the polyurethane resin is a water based aliphatic polyurethane with an acid group functionality.

The following are further examples of the denture composition of the invention:

### Example 3

| | |
|---|---|
| Acetone | 70% (70 grams) |
| 5-methyl-2-pyrrolidinone | 5% (5 grams) |
| Polychlorotrifluorethylene | 15% (15 grams) |
| 2,4 TDI/ED/PEO | 10% (10 grams) |

### Example 4

| | |
|---|---|
| Isopropylalcohol | 80% (80 grams) |
| 1-ethyl-2-pyrrolidinone | 12% (12 grams) |
| Polyvinylidine fluoride | 5% (5 grams) |
| MDI/EG/PPO | 3% (3 grams) |

### Example 5

| | |
|---|---|
| Isopropylacetone | 85% (85 grams) |
| 2-Pyrrolidone | 3% (3 grams) |
| Polyvinylfluoride | 9% (9 grams) |
| MDI/ED/PEO | 3% (3 grams) |

In order to use the composition of the invention on dentures, the dentures are first removed and then cleaned and dried. Thereafter, the composition is preferably sprayed on all external surfaces of the denture by means of a pump or aerosol delivery device. Spraying should take place evenly for between approximately 2 and 7 seconds.

After spraying, the dentures are air dried for at least one hour, and preferably for about two hours. From a practical standpoint, the spraying of the dentures with the dental composition should take place prior to the denture wearer going to bed so that the dentures are sufficiently dry after the spraying process is completed. If that is not convenient, then the sprayed dentures may be rapidly dried by using a hair dryer or other similar drying or air source.

For most denture wearers, application of the composition to one's dentures is required only once a week. If the composition of the invention is properly applied, the accumulation of plaque and other debris along the dentures' surfaces is substantially prevented. As a result, routine cleaning of the treated dentures is quicker and more efficient.

Moreover, it has been found that the composition of the invention helps maintain retention of the dentures in the wearer's mouth. Furthermore, use of the denture composition reduces mouth odors of the denture wearer.

Although the composition of the invention has been described as being suitable for spraying onto a denture material, the composition may be applied to dentures or other artificial teeth in other ways, such as dipping or painting.

For application to a dental implant, the composition is more concentrated than the composition preferred for application to dentures, and preferably includes a solvent in an amount between about 46 and 76 weight percent, a volatile compound such an N-methylpyrrolidinone in an amount between 5 and 25 weight percent, a polyurethane resin in an amount between 10 and 35 weight percent and a poly(fluoro) compound such as polytetra-fluorethylene (Teflon) in an amount between 5 and 20 weight percent.

Preferably, the solvent is present in an amount between 50 and 66 weight percent. The preferred volative compound is N-methylpyrrolidinone in an amount between 9 and 20 weight percent.

If the solvent is either water or an alcohol, as stated hereinabove, a water based aliphatic polyurethane resin is chosen and is present in an amount between 5 and 25 weight percent. If instead a ketone is used as a solvent, a non-water based aromatic resin is chosen and is present in an amount between 5 and 25 weight percent.

Polytetra-fluoroethylene is the preferred hydrocarbon and is present in an amount between 5 and 15 weight percent.

In order to better understand the composition of the invention as applied to a dental implant, the following preferred examples are provided:

### Example 1

| | |
|---|---|
| Water | 66 weight percent (66 grams) |
| N-Methylpyrrolidinone | 9 weight percent (9 grams) |
| Polyurethane | 20 weight percent (20 grams) |
| Polytetra-fluorethylene | 5 weight percent (5 grams) |

### Example 2

| | |
|---|---|
| Water | 64 weight percent (64 grams) |
| Ethanol | 2 weight percent (2 grams) |
| N-Methylpyrrolidinone | 9 weight percent (9 grams) |
| Polytetra-fluorethylene | 5 weight percent (5 grams) |
| Polyurethane | 20 weight percent (20 grams) |

### Example 3

| | |
|---|---|
| Acetone | 70 weight percent (70 grams) |
| 5-methyl-2-pyrrolidinone | 5 weight percent (5 grams) |
| Polychlorotrifluorethylene | 15 weight percent (15 grams) |
| 2,4 TDI/ED/PPO | 10 weight percent (10 grams) |

### Example 4

| | |
|---|---|
| Isopropylalcohol | 60 weight percent (60 grams) |
| 1-ethyl-2-pyrrolidinone | 12 weight percent (12 grams) |
| Polyvinylidine fluoride | 15 weight percent (15 grams) |
| MDI/EG/PPO | 13 weight percent (13 grams) |

### Example 5

| | |
|---|---|
| Isopropylacetone | 55 weight percent (55 grams) |
| 2-Pyrrolidone | 13 weight percent (13 grams) |
| Polyvinylfluoride | 9 weight percent (9 grams) |
| MDI/ED/PEO | 23 weight percent (23 grams) |

### Example 6

| | |
|---|---|
| 1-pentanol | 68 weight percent (68 grams) |
| Diethylether | 12 weight percent (12 grams) |
| Polyvinylidine fluoride | 10 weight percent (10 grams) |
| NDI/ED/PEO | 5 weight percent (5 grams) |

In order to use the composition of the invention on a dental implant, the implant neck is removed (by the dentist) and then cleaned and dried. Thereafter, the neck of the implant is soaked in the inventive composition for about 10-30 seconds. After dipping, the implant neck is air dried for at least two hours.

Alternatively, the inventive composition may be applied to the implant neck by painting.

For most implants, application of the more concentrated form of the inventive composition should be about once every three months. If the composition is properly applied, the accumulation of plaque and other debris along the neck of the implant is substantially prevented. Moreover, there would no longer be the need to scrape and thereby possibly damage the implant surface.

It will thus be seen that the objects set forth above, and those made apparent from the preceding description, are efficiently attained, and since certain changes may be made in carrying out the above method and in the composition set forth above without departing from the spirit and scope of the invention, it is intended that all matter contained in the above description shall be interpreted as illustrative and not in a limiting sense.

It is also to be understood that the following claims are intended to cover all of the generic and specific features of the invention herein described and all statements of the scope of the invention which, as a matter of language, might be said to fall therebetween.

## Claims

1. A method for preventing accumulation or build up of plaque and other debris on dentures or other artificial teeth and dental implants comprising applying to the artificial teeth or implant a composition containing:
a solvent in an amount between 46 and 98 weight percent;
a volatile compound in an amount between 0.5 and 25 weight percent;
a polyurethane-resin in an amount between 1 and 35 weight percent; and
a poly(fluoro) compound in an amount between 0.5 and 20 weight percent.

2. A method as claimed in claim 1, wherein the solvent is water, an alcohol or a ketone.

3. A method as claimed in claim 2, wherein the solvent is ethanol, methanol, isopropyl alcohol, 3-pentanol, 2-pentanol, 1-pentanol, 1-hexanol, 2-hexanol, 3-hexanol isobutyl alcohol, methylethyl ketone, acetone, allylacetone, isopropyl acetone, methylpropyl ketone, 3-pentanone, 3-hexanone or 2-hexanone.

4. A method as claimed in claim 3, wherein the solvent comprises ethanol or methylethyl ketone in an amount between 50 and 97 weight percent.

5. A method as claimed in any one of the preceding claims wherein the volatile compound is a pyrrolidone based material or an ether based material.

6. A method as claimed in claim 5, wherein the volatile compound is N-methylpyrrolidinone, 2-pyrrolidone, 2-pyrrolidoneacetamide, 1-ethyl-2-pyrrolidinone, 5-methyl-2 -pyrrolidinone, diethylether, isopropylether or pentylether.

7. A method as claimed in claim 6, wherein the volatile compound is N-methylpyrrolidinone in an amount between 1 and 20 weight percent.

8. A method as claimed in any one of the preceding calims wherein the polyurethane resin is a water-based aliphatic polyurethane resin or a non-water based aromatic polyurethane resin.

9. A method as claimed in claim 8, wherein the solvent is water or an alcohol and the resin is water based, or is a ketone and wherein the resin is not water based.

10. A method as claimed in any one of the preceding claims, wherein the poly(fluoro) compound is a fluorinated hydrocarbon selected from polytetra-fluorethylene, polychlorotrifluoroethylene, polyhexafluoropropylene, polyvinylidine fluoride, polycinylfluoride, copolymer mixtures of tetra-fluoroethylene, propylene and fluorinated copolymers of ethylene propylene.

11. A method as claimed in claim 10, wherein the fluorinated hydrocarbon is polytetra-fluorethylene in an amount between 0.5 and 15 weight percent.

12. A method as claimed in any one of the preceding claims, wherein the volatile compound is N-methylpyrrolidinone and the poly(fluoro) compound is polytetra-fluorethylene.

13. A method as claimed in any one of the preceding claims wherein the composition is applied to the teeth or implant, optionally after cleaning, by spraying, soaking or painting, and the teeth or implant, and optionally, then dried.

14. A method as claimed in claim 13, wherein drying is effected by air drying the artificial teeth or dental implant for between one and two hours or by drying the artificial teeth or dental implant using a heat or air source.

15. A method as claimed in any one of the preceding claims applied to dentures or other artificial teeth and in which the composition comprises 66 - 98 weight percent of solvent; 0.5 to 15 weight percent of volatile composition; from 1 to 25 weight percent of polyurethane resin; and from 0.5 to 20 weight percent of poly(fluoro) compound.

16. A method as claimed in claim 15, wherein the solvent comprises ethanol or methylethyl ketone in an amount between 75 and 97 weight percent.

17. A method as claimed in claim 15 wherein the volatile material is N-methylpyrrolidinone in an amount between 1 and 8 weight percent.

18. A method as claimed in claim 15 wherein the poly(fluoro)compound is polytetra-fluoroethylene in an amount between 0.5 and 3 weight percent.

19. A method as claimed in any one of claims 15-18, wherein the solvent is present in an amount of about 96 weight percent, N-methylpyrrolidinone is present in an amount of about 1 weight percent , the polyurethane resin is present in an amount of about 2.4 weight percent and polytetrafluoethylene is present in amount of about 0.6 weight percent.

20. A method as claimed in any one of claims 1-14 applied to dental implants and wherein the composition contains from 46 to 76 weight percent of solvent; from 5 to 25 weight percent of volatile compound; from 10 to 35 weight percent of polyurethane resin; and from 0.5 to 20 weight percent of poly(fluoro) compound.

21. A method as claim in claim 20, wherein the solvent comprises ethanol or methylethyl ketone in an amount between 50 and 66 weight percent.

22. A method as claimed in claim 20, wherein the volatile material is N-methylpyrrolidinone in an amount between 9 and 20 weight percent.

23. A method as claimed in claim 20 wherein the poly(fluoro) compound is polytetra-fluorethylene in an amount between 5 and 15 weight percent.

24. A method as claim in claim 20, wherein the solvent is present in an amount of about 66 weight percent, N-methylpyrrolidinone is present in an amount of about 9 weight percent, the polyurethane resin is present in an amount of about 20 weight percent and polytetra-fluorethylene is present in an amount of about 5 weight percent.

## Patentansprüche

1. Verfahren zur Verhinderung der Ansammlung oder des Aufbaus von Plaque und anderen Gewebsresten auf Zahnprothesen oder anderen künstlichen Zähnen und Zahnimplantaten, umfassend das Aufbringen einer Masse auf die künstlichen Zähne oder das Implantat. enthaltend:
ein Lösungsmittel in einer Menge zwischen 46 und 98 Gew.-%;
eine flüchtige Verbindung in einer Menge zwischen 0.5 und 25 Gew.-%;
ein Polyurethanharz in einer Menge zwischen 1 und 35 Gew.%; und
eine Polyfluorverbindung in einer Menge zwischen 0,5 und 20 Gew.%.

2. Verfahren nach Anspruch 1, bei dem das Lösungsmittel Wasser, ein Alkohol oder ein Keton ist.

3. Verfahren nach Anspruch 2, bei dem das Lösungsmittel Ethanol. Methanol, Isopropylalkohol, 3-Pentanol, 2-Pentanol, 1-Pentanol, 1-Hexanol, 2-Hexanol, 3-Hexanol, Isobutylalkohol, Methylethylketon, Aceton, Allylaceton, Isopropylaceton, Methylpropylketon, 3-Pentanon, 3-Hexanon oder 2-Hexanon ist.

4. Verfahren nach Anspruch 3, bei dem das Lösungsmittel Ethanol oder Methylethylketon in einer Menge zwischen 50 und 97 Gew.-% umfaßt.

5. Verfahren nach einem der vorhergehenden Ansprüche, bei dem die flüchtige Verbindung ein Material auf Pyrrolidonbasis oder ein Material auf Etherbasis ist.

6. Verfahren nach Anspruch 5, bei dem die flüchtige Verbindung N-Methylpyrrolidinon, 2-Pyrrolidon, 2-Pyrrolidonacetamid, 1-Ethyl-2-pyrrolidinon, 5-Methyl-2-pyrrolidinon, Diethylether, Isopropylether oder Pentylether ist.

7. Verfahren nach Anspruch 6, bei dem die flüchtige Verbindung N-Methylpyrrolidinon in einer Menge zwischen 1 und 20 Gew.-% ist.

8. Verfahren nach einem der vorhergehenden Ansprüche, bei dem das Polyurethanharz ein wasserlösliches aliphatisches Polyurethanharz oder ein nichtwasserlösliches aromatisches Polyurethanharz ist.

9. Verfahren nach Anspruch 8. bei dem das Lösungsmittel Wasser oder ein Alkohol ist und das Harz wasserlöslich ist oder ein Keton ist und bei dem das Harz nichtwasserlöslich ist.

10. Verfahren nach einem der vorhergehenden Ansprüche, bei dem die Polyfluorverbindung ein fluorierter Kohlenwasserstoff ist, ausgewählt aus Polytetrafluorethylen, Polychlortrifluorethylen, Polyhexafluorpropylen, Polyvinylidenfluorid, Polyvinylfluorid, Copolymerengemischen von Tetrafluorethylen, Propylen und fluorierten Ethylen-Propylen-Copolymeren.

11. Verfahren nach Anspruch 10, bei dem der fluorierte Kohlenwasserstoff Polytetrafluorethylen in einer Menge zwischen 0.5 und 15 Gew.-% ist.

12. Verfahren nach einem der vorhergehenden Ansprüche, bei dem die flüchtige Verbindung N-Methylpyrrolidinon ist und die Polyfluorverbindung Polytetrafluorethylen ist.

13. Verfahren nach einem der vorhergehenden Ansprüche, bei dem die Masse, gegebenenfalls nach dem Säubern, durch Sprühen, Tränken oder Streichen auf die Zähne oder das Implantat aufgebracht wird und die Zähne oder das Implantat dann gegebenenfalls getrocknet werden.

14. Verfahren nach Anspruch 13, bei dem das Trocknen durch Lufttrocknen der künstlichen Zähne oder des Zahnimplantats für zwischen einer und zwei Stunden oder durch Trocknen der künstlichen Zähne oder des Zahnimplantats unter Verwendung einer Wärme- oder Luftquelle bewirkt wird.

15. Verfahren nach einem der vorhergehenden Ansprüche, angewendet auf Zahnprothesen oder andere künstliche Zähne, bei dem die Masse 66-98 Gew.-% Lösungsmittel; 0.5 bis 15 Gew.-% flüchtige Zusammensetzung; von 1 bis 25 Gew.-% Polyurethanharz und von 0.5 bis 20 Gew.-% Polyfluorverbindung umfaßt.

16. Verfahren nach Anspruch 15, bei dem das Lösungsmittel Ethanol oder Methylethylketon in einer Menge zwischen 75 und 97 Gew.-% umfaßt.

17. Verfahren nach Anspruch 15, bei dem das flüchtige Material N-Methylpyrrolidinon in einer Menge zwischen 1 und 8 Gew.-% ist.

18. Verfahren nach Anspruch 15, bei dem die Polyfluorverbindung Polytetrafluorethylen in einer Menge zwischen 0.5 und 3 Gew.-% ist.

19. Verfahren nach einem der Ansprüche 15-18, bei dem das Lösungsmittel in einer Menge von etwa 96 Gew.-% vorliegt, N-Methylpyrrolidinon in einer Menge von etwa 1 Gew.-% vorliegt, das Polyurethanharz in einer Menge von etwa 2,4 Gew.-% vorliegt und das Polytetrafluorethylen in einer Menge von etwa 0.6 Gew.-% vorliegt.

20. Verfahren nach einem der Ansprüche 1-14, angewendet auf Zahnimplantate, und bei dem die Masse von 46 bis 76 Gew.-% Lösungsmittel enthält; von 5 bis 25 Gew.-% flüchtige Verbindung: von 10 bis 35 Gew.-% Polyurethanharz; und von 0.5 bis 20 Gew.-% Polyfluorverbindung.

21. Verfahren nach Anspruch 20, bei dem das Lösungsmittel Ethanol oder Methylethylketon in einer Menge zwischen 50 und 66 Gew.-% umfaßt.

22. Verfahren nach Anspruch 20, bei dem das flüchtige Material N-Methylpyrrolidinon in einer Menge zwischen 9 und 20 Gew.-% ist.

23. Verfahren nach Anspruch 20, bei dem die Polyfluorverbindung Polytetrafluorethylen in einer Menge zwischen 5 und 15 Gew.-% ist.

24. Verfahren nach Anspruch 20. bei dem das Lösungsmittel in einer Menge von etwa 66 Gew.-% vorliegt, N-Methylpyrrolidinon in einer Menge von etwa 9 Gew.-% vorliegt, das Polyurethanharz in einer Menge von etwa 20 Gew.-% vorliegt und Polytetrafluorethylen in einer Menge von etwa 5 Gew.-% vorliegt.

## Revendications

1. Procédé pour la prévention de l'accumulation ou de la formation de plaque et d'autres débris sur les prothèses dentaires ou autres dents artificielles et implants dentaires comprenant l'application sur les dents artificielles ou sur un implant d'une composition contenant:
un solvant en une quantité comprise entre 46 et 98 pourcent en poids;
un composé volatil en une quantité comprise entre 0,5 et 25 pourcent en poids;
une résine de polyuréthane en une quantité comprise entre 1 et 35 pourcent en poids; et
un composé poly(fluoré) en une quantité comprise entre 0,5 et 20 pourcent en poids.

2. Procédé tel que revendiqué dans la revendication 1, dans lequel le solvant est l'eau, un alcool ou une cétone.

3. Procédé tel que revendiqué dans la revendication 2, dans lequel le solvant est l'éthanol, le méthanol, l'alcool isopropylique, le 3-pentanol, le 2-pentanol, le 1-pentanol, le 1-hexanol, le 2-hexanol, le 3-hexanol, l'alcool isobutylique, la méthyléthyl cétone, l'acétone, l'allyl acétone, l'isopropyl acétone, la méthylpropyl cétone, la 3-pentanone, la 3-hexanone ou la 2-hexanone.

4. Procédé tel que revendiqué dans la revendication 3, dans lequel le solvant comprend l'éthanol ou la méthyléthyl cétone en une quantité comprise entre 50 et 97 pourcent en poids.

5. Procédé tel que revendiqué dans l'une quelconque des revendications précédentes, dans lequel le composé volatil est une substance à base de pyrrolidone ou une substance à base d'éther.

6. Procédé tel que revendiqué dans la revendication 5, dans lequel le composé volatil est la N-méthylpyrrolidinone, la 2-pyrrolidone, le 2-pyrrolidoneacétamide, la 1-éthyl -2-pyrrolidinone, la 5-méthyl -2-pyrrolidinone, l'éther diéthylique, l'éther isopropylique ou l'éther pentylique.

7. Procédé tel que revendiqué dans la revendication 6, dans lequel le composé volatil est la N-méthylpyrrolidinone en une quantité comprise entre 1 et 20 pourcent en poids.

8. Procédé tel que revendiqué dans l'une quelconque des revendications précédentes, dans lequel la résine de polyuréthane est une résine de polyuréthane aliphatique à base aqueuse ou une résine de polyuréthane aromatique à base non-aqueuse.

9. Procédé tel que revendiqué dans la revendication 8, dans lequel le solvant est l'eau ou un alcool et la résine est à base aqueuse, ou le solvant est une cétone et dans lequel la résine n'est pas à base aqueuse.

10. Procédé tel que revendiqué dans l'une quelconque des revendications précédentes, dans lequel le composé poly(fluoré) est un hydrocarbure fluoré choisi parmi le polytétrafluoroéthylène, le polychlorotrifluoroéthylène, le polyhexafluoropropylène, le polyfluorure de vinylidène, le polyfluorure de vinyle, les mélanges copolymères de tétrafluoroéthylène propylène et les copolymères fluorés d'éthylène-propylène.

11. Procédé tel que revendiqué dans la revendication 10, dans lequel l'hydrocarbure fluoré est le polytétrafluoroéthylène en une quantité comprise entre 0,5 et 15 pourcent en poids.

12. Procédé tel que revendiqué dans l'une quelconque des revendications précédentes, dans lequel le composé volatil est la N-méthylpyrrolidinone et le composé poly(fluoré) est le polytétrafluoroéthylène.

13. Procédé tel que revendiqué dans l'une quelconque des revendications précédentes, dans lequel la composition est appliquée sur les dents ou sur l'implant, éventuellement après nettoyage, par pulvérisation, trempage ou peinture, et les dents ou l'implant sont ensuite et éventuellement séchés.

14. Procédé tel que revendiqué dans la revendication 13, dans lequel le séchage est effectué par séchage à l'air des dents artificielles ou de l'implant dentaire pendant une durée comprise entre une et deux heures ou par séchage des dents artificielles ou de l'implant dentaire à l'aide d'une source de chaleur ou d'air.

15. Procédé tel que revendiqué dans l'une quelconque des revendications précédentes appliqué à une prothèse dentaire ou à d'autres dents artificielles et dans lequel la composition comprend de 66 à 98 pourcent en poids de solvant; de 0,5 à 15 pourcent en poids de composition volatile; de 1 à 25 pourcent en poids de résine de polyuréthane; et de 0,5 à 20 pourcent en poids de composé poly(fluoré).

16. Procédé tel que revendiqué dans la revendication 15, dans lequel le solvant comprend l'éthanol ou la méthyléthyl cétone en une quantité comprise entre 75 et 97 pourcent en poids.

17. Procédé tel que revendiqué dans la revendication 15, dans lequel le composé volatil est la N-méthylpyrrolidinone en une quantité comprise entre 1 et 8 pourcent en poids.

18. Procédé tel que revendiqué dans la revendication 15, dans lequel le composé poly(fluoré) est le polytétrafluoroéthylène en une quantité comprise entre 0,5 et 3 pourcent en poids.

19. Procédé tel que revendiqué dans l'une quelconque des revendications 15 à 18, dans lequel le solvant est présent en une quantité d'environ 96 pourcent en poids, la N-méthylpyrrolidinone est présente en une quantité d'environ 1 pourcent en poids, la résine de polyuréthane est présente en une quantité d'environ 2,4 pourcent en poids et le polytétrafluoroéthylène est présent en une quantité d'environ 0,6 pourcent en poids.

20. Procédé tel que revendiqué dans l'une quelconque des revendications 1-14 appliqué à des implants dentaires et dans lequel la composition contient de 46 à 76 pourcent en poids de solvant; de 5 à 25 pourcent en poids de composé volatil; de 10 à 35 pourcent en poids de résine de polyuréthane; et de 0,5 à 20 pourcent en poids de composé poly(fluoré).

21. Procédé tel que revendiqué dans la revendication 20, dans lequel le solvant comprend l'éthanol ou la méthyléthyl cétone en une quantité comprise entre 50 et 66 pourcent en poids.

22. Procédé tel que revendiqué dans la revendication 20, dans lequel le produit volatil est la N-méthylpyrrolidinone en une quantité comprise entre 9 et 20 pourcent en poids.

23. Procédé tel que revendiqué dans la revendication 20, dans lequel le composé poly(fluoré) est le polytétrafluoroéthylène en une quantité comprise entre 5 et 15 pourcent en poids.

24. Procédé tel que revendiqué dans la revendication 20, dans lequel le solvant est présent en une quantité d'environ 66 pourcent en poids, la N-méthylpyrrolidinone est présente en une quantité d'environ 9 pourcent en poids, la résine de polyuréthane est présente en une quantité d'environ 20 pourcent en poids et le polytétrafluoroéthylène est présent en une quantité d'environ 5 pourcent en poids.
